(19) Europäisches Patentamt
European Patent Office
Office européen des brevets

(11) Veröffentlichungsnummer : **0 135 070**
**B1**

# (12) EUROPÄISCHE PATENTSCHRIFT

(45) Veröffentlichungstag der Patentschrift : **23.03.88**

(51) Int. Cl.⁴ : **C 12 N 9/06** // (C12N9/06, C12R1:38)

(21) Anmeldenummer : **84108877.6**

(22) Anmeldetag : **26.07.84**

## (54) Sarcosin-Oxidase.

(30) Priorität : **26.07.83 DE 3326888**

(43) Veröffentlichungstag der Anmeldung : **27.03.85 Patentblatt 85/13**

(45) Bekanntmachung des Hinweises auf die Patenterteilung : **23.03.88 Patentblatt 88/12**

(84) Benannte Vertragsstaaten :
**AT BE CH DE FR GB IT LI LU NL SE**

(56) Entgegenhaltungen :
**DE-A- 2 842 940**
**BIOCHEMICAL AND BIOPHYSICAL RESEARCH COMMUNICATIONS, Band 46, Nr. 2, 1972, Seiten 885-891, Academic Press, Inc.; D.R. PATEK et al.: "Isolation of acid-nonextractable flavins from a bacterial sarcosine oxidase"**
**CHEMICAL ABSTRACTS, Band 74, Nr. 17, April 1971, Seite 140, Nr. 95794b, Columbus, Ohio, US; W.R. FRISELL: "One-carbon metabolism in microorganisms. I. Oxidative demethylation in a sarcosine-utilizing bacterium"**

(73) Patentinhaber : **BOEHRINGER MANNHEIM GMBH**
**Patentabteilung, Abt. E Sandhofer Strasse 112-132**
**Postfach 31 01 20**
**D-6800 Mannheim 31 Waldhof (DE)**

(72) Erfinder : **Mayr, Ulrich, Dr. rer. nat.**
**Pürstlingstrasse 21**
**D-8200 Rosenheim (DE)**
Erfinder : **Gauhl, Helmgard**
**Mitterfeld 4**
**D-8132 Tutzing (DE)**
Erfinder : **Seidel, Hans, Dr. rer. nat.**
**Waxensteinstrasse 6**
**D-8132 Tutzing (DE)**
Erfinder : **Röder, Albert, Dr. rer. nat.**
**Tiefentalweg 8**
**D-8124 Seehaupt (DE)**

(74) Vertreter : **Weickmann, Heinrich, Dipl.-Ing. et al**
**Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel**
**Postfach 860820**
**D-8000 München 86 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die Erfindung betrifft ein Verfahren zur Gewinnung von Sarcosin-Oxidase aus Mikroorganismen.

Sarcosin-Oxidase E.C. 1.5.3.1 ist bekannt und wurde bereits aus Mikroorganismen wie Corynebakterien, Arthrobacter, Bacillus und Cylindrocarpon gewonnen. Das Enzym ist technisch von Interesse im Rahmen der Bestimmung von Sarcosin oder von Verbindungen, die in Sarcosin überführbar sind. Es katalysiert die Reaktion

$$\text{Sarcosin} + O_2 + H_2O \longrightarrow HCHO + NH_2CH_2COOH + H_2O_2.$$

Die Reaktionsprodukte, insbesondere $H_2O_2$, lassen sich nach bekannten Methoden leicht bestimmen.

Ein Hemmnis für den Einsatz der Sarcosin-Oxidase in einem derartigen Bestimmungsverfahren liegt darin, daß eine Gewinnung aus den Mikroorganismen aufwendig ist, da alle Mikroorganismen, in denen das Enzym bisher in einer die Aufarbeitung lohnenden Menge gefunden wurde, als relativ anspruchsvoll, insbesondere hinsichtlich ihrer Kultivierbarkeit, anzusehen sind.

Der Erfindung liegt daher die Aufgabe zugrunde, ein Verfahren zur Gewinnung der Sarcosin-Oxidase aus leicht kultivierbaren, anspruchslosen, ubiquitär vorkommenden Mikroorganismen aufzufinden.

Diese Aufgabe wird gelöst durch ein Verfahren zur Gewinnung von Sarcosin-Oxidase aus Mikroorganismen durch Züchtung derselben und Gewinnung des Enzyms aus der Biomasse oder der Kulturbrühe, welches dadurch gekennzeichnet ist, daß man einen Pseudomonas-Stamm züchtet.

Es ist überraschend, daß Pseudomonaden Sarcosin-Oxidase enthalten, da bisher in diesen Mikroorganismen nur das Vorkommen von Sarcosin-Dehydrogenase E.C. 1.5.99.1 bekannt war (Agric. Biol. Chem. 43, 1197-1203 (1979)). Daher war nicht zu erwarten, daß auch eine Sarconsin-Oxidase in diesen Mikroorganismen, die sich durch die oben angegebenen Eigenschaften, also leichte Kultivierbarkeit, Anspruchslosigkeit und weit verbreitetes Vorkommen auszeichnen, zu finden sein würde.

Ein besonderer Vorteil der Erfindung liegt darin, daß sehr hohe Aktivitäten an Sarcosin-Oxidase erzielbar sind und Stämme gefunden werden konnten, bei denen bis zu 20 % ihres löslichen Proteins aus Sarcosin-Oxidase (Sar-OD) bestehen. Bevorzugt werden im Rahmen der Erfindung die folgenden Pseudomonas-Stämme verwendet :

Pseudomonas maltophilia (BMTU 254) DSM 2700,

Pseudomonas maltophilia (BMTU 288) DSM 2701,

Die erfindungsgemäß verwendeten Pseudomonas-Stämme lassen sich höchst einfach auf einem Creatin oder Sarcosin als Kohlenstoff- und Stickstoffquelle sowie Salzen und Spurenelementen enthaltenden Medium züchten.

Ein geeignetes Minimal-Medium besteht beispielsweise aus Creatin bzw. Sarcosin, $MgSO_4$, NaCl, $NH_4Cl$, $KH_2PO_4$ und $Na_2HPO_4$. Es eignet sich besonders zur Selektion besonders Sar-OD-reicher Stämme. Eine geeignete Lösung von Spurenelementen enthält Mn, Fe, Ca, Cu, Zn, Mo und Co. Für die Produktion läßt man die Mikroorganismen vorzugsweise auf einem Medium wachsen, welches neben Creatin oder Sarcosin noch Hefeextrakt, Trypton und NaCl enthält.

Besonders hohe Ausbeuten lassen sich erzielen, wenn man den Mikroorganismus mit Nitrosoguanidin inkubiert und anschließend auf dem obengenannten Minimalmedium weiter züchtet, wobei die besonders gut wachsenden Kolonien isoliert und weitergezüchtet werden.

Das erfindungsgemäß erhältliche Enzym weist folgende Eigenschaften auf :

pH-Optimum : 8,0

Temperaturstabilität 10 Minuten bei 37 °C ; pH 8,0. 84 % Restaktivität.

10 Minuten 45 °C 13 %

$K_M$ Sarcosin, pH 8,0 ; 0,1 M Trispuffer, 25 °C ; PAP-Nachweissystem : $4 \times 10^{-3}$ M.

Die Isolierung des Enzyms kann nach den üblichen Methoden der Enzymreinigung erfolgen, beispielsweise wie in Biochem. Biophys. Res. Comm. Vol. 96, 924-930 (1980) beschrieben. Bevorzugt wird jedoch ein Anreicherungsverfahren, bei dem nach Aufschluß, beispielsweise in der Hochdruckdispersion, eine Polyäthyleniminfraktionierung durchgeführt wird und die erhaltene aktive Fraktion über hydrophobe Phenylsepharose chromatographiert wird. Anschließend erfolgt eine Molekularsiebfraktionierung und eine Feinreinigung an Oktylsepharose. Nach diesem Verfahren läßt sich mit einer Ausbeute von über 30 % ein Präparat mit einer spezifischen Aktivität zwischen 8 und 9 U/mg erhalten.

Die Mikroorganismen werden nach der Ernte vorzugsweise in einer Pufferlösung vom pH 6 bis 9,5, welche 0,1 bis 0,3 Gew.-% Polyäthylenimin enthält, mechanisch aufgeschlossen. Man trennt das Unlösliche ab und verdünnt den so erhaltenen Aufschlußüberstand auf einen Polyäthylenimingehalt von 0,05 Gew.-% oder weniger mit Wasser oder Pufferlösung, wobei die Sar-OD ausfällt und als Niederschlag gewonnen wird.

Für die Feinreinigung des so erhaltenen Enzympräparates wird das Präparat zweckmäßig wieder in Pufferlösung aufgenommen, über hydrophobe Phenylsepharose fraktioniert, zweckmäßig mit absteigendem Gradienten, die aktive Fraktion wird mit Ammoniumsulfat fraktioniert und die zwischen 1,8 und 2,3 M Ammoniumsulfat ausfallende Fraktion einer Molekularsiebpassage, beispielsweise an vernetzter Agarose,

unterzogen und schließlich über Octylsepharose chromatographiert. Man erhält so das reine Enzym, welches eine spezifische Aktivität von etwa 8,4 U/mg aufweist.

Für einen Einsatz des Enzyms für analytische Zwecke ist die Feinreinigung nicht oder nicht in vollem Umfange erforderlich. Falls das mit Polyäthylenimin gewonnene Rohenzym für die Bestimmung noch zu viel Nebenaktivitäten enthält, genügt in der Regel die Fraktionierung über Phenylsepharose und mit Ammoniumsulfat, um ausreichende Reinheit zu erzielen.

Das erfindungsgemäß erhältliche Enzym entspricht in seinen Eigenschaften dem bekannten Enzym und bedarf daher hier keiner näheren Beschreibung. Durch das erfindungsgemäße Verfahren läßt sich das Enzym auf einfache Weise in hohen Ausbeuten gewinnen und kann beispielsweise für die Bestimmung von Sarcosin oder in Sarcosin überführbaren Verbindungen wie Creatinin in biologischen Flüssigkeiten verwendet werden.

Die folgenden Beispiele erläutern die Erfindung weiter :

## Beispiel 1

Die Pseudomonas-Stämme wurde in einem Minimal-Medium der folgenden Zusammensetzung ca. 20 Stunden bei 30 °C fermentiert :

pro Liter :
7,0 g $Na_2HPO_4 \cdot 2H_2O$
3,0 g $KH_2PO_4$
1,0 g $NH_4Cl$
0,05 g NaCl
5 ml $MgSO_4 \cdot 7H_2O$* 10 proz. (nach Sterilisation zugeben)
1 ml Spurenlösung
10 g Creatin bzw. Sarcosin (getrennt sterilisiert)

pH 7
* Spurenlösung pro 100 ml :
0,1 g $MnSO_4 \cdot 2H_2O$-0,1 g $FeSO_4 \cdot 7H_2O$-0,1 g $CaCl_2 \cdot 2H_2O$-0,01 g $CuSO_4 \cdot 5H_2O$-0,01 g $ZnCl_2$-0,01 g $(NH_4)_2MoO_4$-0,01 g $CoSO_4 \cdot 7H_2O$

Dabei wurden folgende Sar-OD-Aktivitäten durch Bestimmung von gebildetem $H_2O_2$ gemessen :

| | BMTU-Nr. | korresp. Nr. | Sar-OD Aktivität, u/l Kulturbr. |
|---|---|---|---|
| Pseudomonas maltophilia | 254 | DSM 2700 | 30 |
| Pseudomonas Maltophilia | 288 | DSM 2701 | 25 |

## Beispiel 2

Pseudomonas maltophilia (BMTU 254) DSM 2700 wurde aerob in LB-Medium (1 % Difco-Trypton, 0,5 % Difco-Hefeextrakt, 0,5 % NaCl, pH 7,2) bei 28 °C bis zu einer OD 400 bis 600 nm = 1 angezogen. Die Zellen wurden in 0,1 M Citratpuffer pH 5,5 gewaschen, in demselben Puffer mit Nitrosoguanidin (80 ug/ml) 60 Minuten bei 30 °C inkubiert und danach mit 0,1 M Phosphatpuffer pH 7,0 gewaschen. Anschließend erfolgte eine dreistündige aerobe Anzucht in LB-Medium bei 28 °C. Die Zellen wurden erneut in obigem Phosphatpuffer gewaschen und auf Minimalmedium (0,6 % $Na_2HPO_4$, 0,3 % $KH_2PO_4$, 0,05 % NaCl, 1 mM $MgSO_4$, 0,1 mM $CaCl_2$, 1 ml Spurenlösung [1)]/1 Medium) mit 0,5 % Kreatin bzw. Sarkosin als Kohlenstoff- und Stickstoffquelle ausplattiert. Die im Vergleich zum Ausgangsstamm besser wachsenden Mutanten

[1)] Die Spurenlösung enthält/100 ml
0,007 g $FeSO_4 \times 7H_2O$
0,015 g $MnCl_2 \times 4H_2O$
0,015 g $ZnSO_4 \times 7H_2O$
0,0015 g $CuSO_4 \times 5H_2O$
0,0015 g $CaCl_2 \times 6H_2O$
0,0015 g $NaMoO_4 \times 2H_2O$
0,0015 g $NiCl_2 \times 6H_2O$

wurden isoliert und nach obigem Prinzip einer weiteren Nitrosoguanidinbehandlung und anschließender Selektion auf Minimalmedium mit 0,5 % Kreatin bzw. Sarkosin unterworfen.

Das aus dieser Selektion hervorgegangene Isolat wurde in einem Medium folgender Zusammensetzung ca. 32 Stunden fermentiert :

1 % Difco-Trypton
0,5 % Difco-Hefeextrakt
0,5 % Sarcosin
0,5 % NaCl
ph 7,1, 25 °C.

Es wurde eine Aktivitätsausbeute von 1200 U/l Kulturlösung erreicht. Im Rohextrakt-Überstand wurde eine spezifische Aktivität der Sarcosin-Oxidase von 1,9 U/mg gemessen ; dies entspricht ca. 22 % des löslichen Proteins.

Beispiel 3

Isolierung von Sarcosin-Oxidase EC 1.5.3.1

2,1 kg Feuchtmasse Pseudomonas maltophilia, BMTU 254, DSM 2700 (resultierend aus 100 Liter Kulturlösung, Beispiel 2) wurden mit 200 mmol/l TRIS-Puffer pH 8,0 suspendiert (18 Liter) und 4 mal bei 650 atü in der Hochdruckdispersionsmaschine aufgeschlossen. Der Aufschluß-Lösung wurde soviel Polimin $G_{35}$ (Fa. BASF) zugegeben, daß eine weitgehende Abtrennung von Nucleinsäure und Fremdproteinen erfolgte (0,2 %). Zur positiven Enzymfällung genügte die Verdünnung mit $H_2O$. Der Sarcosin Oxidase-Niederschlag wurde mit 1,5 Liter 20 mmol/l TRIS, enthaltend 1 mmol/l Ammoniumsulfat pH 8,0, gelöst und dalysiert. Es folgte eine hydrophobe Chromatographie an Phenyl-Sepharose pH 8,0 im absteigenden Gradienten. nach einer Salzfraktionierung mittels Ammoniumsulfat zwischen 1,8 und 2,3 mol/l wurde eine Molekular-Sieb-Passage an Sephrose $S_{200}$ (PHARMACIA) durchgeführt. Der Sarcosin-Oxidase-Peak wurde an Oktyl-Sepharose zwischen pH 6,3 und 7,0 (steigender pH-Gradient) bis 8,3 U/mg Protein gereinigt. Das gereinigte Enzym ist frei von Uricase und Katalase und zeigt in der SDS-Disk Elektrophorese vier verschiedene Untereinheiten, wie das Enzym aus Corynebacterium species 0 96 von Suzuki (J. Biochem. 89, 599-607, 1981). Die Tabelle zeigt die Anreicherung und Ausbeute.

Sarcosin Oxidase-Anreicherung aus 2,1 kg Feuchtmasse Pseudomonas maltophilia

| Schritt | Volumen | Units | Protein | Spez. Akt. | Ausbeute % |
|---|---|---|---|---|---|
| 4x650 Atü | 18 l | $1{,}4 \times 10^5$ | 118 g | 1,19 | 100 |
| $G_{35}$-Polimin Fraktion | 1,5 l | $1{,}2 \times 10^5$ | 53 g | 2,26 | 85,7 |
| Phenyl-Sepharose | 765 ml | $8{,}6 \times 10^4$ | 16,5 g | 5,2 | 61 |
| Gel-Filtration $S_{200}$ | 260 ml | $8{,}0 \times 10^4$ | 11,4 g | 7,0 | 57 |
| Oktylsepharose | 168 ml | $6{,}65 \times 10^4$ | 8,0 g | 8,3 | 47,5 |

**Patentansprüche**

1. Verfahren zur Gewinnung von Sarcosin-Oxidase EC 1.5.3.1 aus Mikroorganismen durch Züchtung derselben und Gewinnung des Enzyms aus der Biomasse oder der Kulturbrühe, dadurch gekennzeichnet, daß man einen Pseudomonas-Stamm züchtet.

2. Verfahren nach Anspruch 1, dadurch gekennzeichnet, daß man Pseudomonas maltophilia (BMTU 254) DSM 2700 oder Pseudomonas maltophilia (BMTU 288) DSM 2701 züchtet.

3. Verfahren nach Anspruch 2, dadurch gekennzeichnet, daß man Pseudomonas-Mutanten verwendet, die aus dem Wildstamm von Anspruch 2 durch Behandlung mit Nitrosoguanidin erhalten werden.

4. Verfahren nach Anspruch 2 oder 3, dadurch gekennzeichnet, daß man Pseudomonas-Mutanten verwendet, die einer Selektion auf Creatin als Kohlenstoff- und Stickstoffquelle unterzogen wurden.

5. Verfahren nach einem der Ansprüche 1 bis 4, dadurch gekennzeichnet, daß man in einem Medium züchtet, welches Creatin oder/und Sarcosin, Trypton, Hefeextrakt und Kochsalz sowie Spurenelemente enthält.

6. Verfahren nach einem der vorhergehenden Ansprüche, dadurch gekennzeichnet, daß man die Mikroorganismenmasse in Pufferlösung pH 6 bis 9,5, welche 0,1 bis 0,3 Gew.-% Polyäthylenimin enthält, mechanisch aufschließt, vom Unlöslichen trennt, den Überstand auf einen Gehalt von höchstens 0,05 % Polyäthylenimin mit Wasser verdünnt und den gebildeten Niederschlag gewinnt.

7. Verfahren nach Anspruch 6, dadurch gekennzeichnet, daß man zur Feinreinigung den Niederschlag in Puffer löst, über hydrophobe Phenylsepharose fraktioniert, die aktive Fraktion mit Ammonsulfat behandelt und die zwischen 1,8 und 3,2 M Ammonsulfat unlösliche Fraktion gewinnt und einer Molekularsiebfraktionierung und einer Chromatographie über Octylsepharose unterwirft.

## Claims

1. Process for the obtaining of sarcosine oxidase EC 1.5.3.1 from micro-organisms by culturing thereof and obtaining of the enzyme from the biomass or the culture broth, characterised in that one cultures a Pseudomonas strain.

2. Process according to claim 1, characterised in that one cultures Pseudomonas maltophilia (BMTU 254) DSM 2700 or Pseudomonas maltophilia (BMTU 288) DSM 2701.

3. Process according to claim 2, characterised in that one uses Pseudomonas mutants which are obtained from the wild strain of claim 2 by treatment with nitrosoguanidine.

4. Process according to claim 2 or 3, characterised in that one uses Pseudomonas mutants which have been subjected to a selection on creatine as carbon and nitrogen source.

5. Process according to one of claims 1 to 4, characterised in that one cultures in a medium which contains creatine and/or sarcosine, trypton, yeast extract and common salt, as well as trace elements.

6. Process according to one of the preceding claims, characterised in that one mechanically digests the micro-organism mass in buffer solution pH 6 to 9.5 which contains 0.1 to 0.3 % wt.% of polyethyleneimine, separates from insolubles, dilutes the supernatant with water to a content of at most 0.05 % polyethylene-imine and recovers the precipitate formed.

7. Process according to claim 6, characterised in that, for the high purification, one dissolves the precipitate in a buffer, fractionates over hydrophobic phenylsepharose, treats the active fraction with ammonium sulphate and obtains the fraction insoluble between 1.8 and 3.2 M ammonium sulphate and subjects to a molecular sieve fractionation and to a chromatography over octylsepharose.

## Revendications

1. Procédé d'obtention de la sarcosine-oxydase E.C. 1.5.3.1. à partir de microorganismes par culture de ceux-ci et obtention de l'enzyme à partir de la biomasse ou du bouillon de culture, caractérisé en ce qu'on cultive une souche de Pseudomonas.

2. Procédé selon la revendication 1, caractérisé en ce qu'on cultive Pseudomonas maltophilia (BMTU 254) DSM 2700 ou Pseudomonas maltophilia (BMTU 288) DSM 2701.

3. Procédé selon la revendication 2, caractérisé en ce qu'on utilise des mutants de Pseudomonas, qui sont obtenus à partir de la souche sauvage de la revendication 2 par traitement par la nitrosoguanidine.

4. Procédé selon les revendications 2 ou 3, caractérisé en ce qu'on utilise des mutants de Pseudomonas, qui ont été soumis à une sélection sur créatine comme source de carbone et d'azote.

5. Procédé selon l'une des revendications 1 à 4, caractérisé en ce qu'on cultive dans un milieu qui contient de la créatine ou/et de la sarcosine, de la tryptone, de l'extrait de levure et du chlorure de sodium ainsi que des oligo-éléments.

6. Procédé selon l'une des revendications précédentes, caractérisé en ce qu'on désagrège mécaniquement la masse de microorganismes dans une solution tampon à pH 6 à 9,5, qui contient 0,1 à 0,3 % de polyéthylènimine, en ce qu'on sépare la matière insoluble, en ce qu'on dilue à l'eau le liquide surnageant jusqu'à une teneur en polyéthylènimine de 0,05 % au maximum et en ce qu'on récupère le précipité formé.

7. Procédé selon la revendication 6, caractérisé en ce qu'on dissout le précipité dans un tampon pour la purification fine, en ce qu'on le fractionne sur Phénylsépharose hydrophobe, en ce qu'on traite la fraction active par le sulfate d'ammonium et en ce qu'on récupère la fraction insoluble dans le sulfate d'ammonium entre 1,8 et 3,2 M et la soumet à un fractionnement sur tamis moléculaire et à une chromatographie sur Octylsépharose.